(19) Europäisches Patentamt
European Patent Office
Office européen des brevets

(11) **EP 2 846 285 B1**

(12) **EUROPEAN PATENT SPECIFICATION**

(45) Date of publication and mention
of the grant of the patent:
**24.04.2019 Bulletin 2019/17**

(51) Int Cl.:
*G16H 50/20* (2018.01)   *G16H 50/30* (2018.01)

(21) Application number: **14183679.1**

(22) Date of filing: **05.09.2014**

(54) **Risk stratification of suspected AMI patients**

Risikostratifizierung von vermuteten AMI-Patienten

Stratification des risques de patients soupçonnés d'être atteint d'infarctus aigu du myocarde

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.09.2013 GB 201315943**

(43) Date of publication of application:
**11.03.2015 Bulletin 2015/11**

(73) Proprietor: **Randox Laboratories Ltd.
Crumlin, County Antrim BT29 4QY (GB)**

(72) Inventor: **McEneaney, David
Craigavon, BT63 5QQ (GB)**

(56) References cited:
• **WILFRIED DINH ET AL: "High sensitive troponin T and heart fatty acid binding protein: Novel biomarker in heart failure with normal ejection fraction?: A cross-sectional study", BMC CARDIOVASCULAR DISORDERS, BIOMED CENTRAL, LONDON, GB, vol. 11, no. 1, 5 July 2011 (2011-07-05), page 41, XP021103208, ISSN: 1471-2261, DOI: 10.1186/1471-2261-11-41**

• **MICHAEL J DALY ET AL: "Heart fatty acid binding protein in combination with the 80-lead body surface potential map improves early detection of acute myocardial infarction in patients who are cardiac troponin T negative at presentation", JOURNAL OF ELECTROCARDIOLOGY, vol. 44, no. 4, 29 November 2011 (2011-11-29), pages 432-438, XP028231105, ISSN: 0022-0736, DOI: 10.1016/J.JELECTROCARD.2011.03.001 [retrieved on 2011-03-04]**

• **EMMACE-2 INVESTIGATORS KILCULLEN ET AL: "Heart-Type Fatty Acid-Binding Protein Predicts Long-Term Mortality After Acute Coronary Syndrome and Identifies High-Risk Patients Across the Range of Troponin Values", JOURNAL OF THE AMERICAN COLLEGE OF CARDIOLOGY, ELSEVIER, NEW YORK, NY, US, vol. 50, no. 21, 5 November 2007 (2007-11-05), pages 2061-2067, XP022418188, ISSN: 0735-1097, DOI: 10.1016/J.JACC.2007.08.021**

• **RICHARD BODY ET AL: "A FABP-ulous rule out strategy? Heart fatty acid binding protein and troponin for rapid exclusion of acute myocardial infarction", RESUSCITATION, ELSEVIER, IE, vol. 82, no. 8, 15 March 2011 (2011-03-15) , pages 1041-1046, XP028240007, ISSN: 0300-9572, DOI: 10.1016/J.RESUSCITATION.2011.03.015 [retrieved on 2011-04-02]**

## Description

## Background

Cardiovascular disease is the largest cause of death in the developed world, with almost 50% of all cardiovascular deaths being due to coronary artery disease. This places a significant economic burden on the European Economic Area. It has been estimated that cardiovascular disease costs the European Economic Area €169 billion per year. Diagnosing coronary artery disease still presents a challenge for the physician even with currently available diagnostic aids. Studies have shown that between 2 and 5% of patients with myocardial infarction (MI) are discharged from accident and emergency departments with the diagnosis missed (Storrow and Gibler, 2000), with rates as high as 11.1% in some cases (Pope *et al.,* 2000).

The simplest and cheapest diagnostic aid is the 12 lead Electrocardiogram (ECG). Changes that may indicate ischaemia or infarction include ST segment elevation or depression, left bundle branch block (LBBB), T wave changes and ultimately pathological Q waves. The initial ECG, however, only yields a sensitivity of between 13 and 70% for a diagnosis of MI (Speake & Terry, 2001). Using multi-lead surface mapping ECG increases sensitivity modestly, but still without sufficient negative predictive value to rule out infarction (Owens et *al.,* 2004; McClelland *et al.,* 2003).

More recently, advanced imaging techniques have been evaluated as to their role in the diagnosis and risk stratification of ACS. Assessment in the Emergency department with novel T2-weighted cardiac magnetic resonance (CMR) protocols may improve detection of acute coronary syndromes but, as yet, cannot reliably distinguish evolving MI from angina without infarction (Cury *et al.,* 2008). Emergency department CMR is also not widely available, and is contraindicated in some patients due to ferrous metal implants, currently making this an impractical solution to ACS diagnosis.

Referring to the universal definition of myocardial infarction it is clear that biomarker assays are now also pivotal to the diagnosis (Thygesen *et al.,* 2012). Currently cardiac troponin measurement (4th generation cardiac troponin T and cardiac troponin I) remains the gold standard biomarker assay of myocardial necrosis. Assays have near 100% tissue specificity and high clinical sensitivity, thereby reflecting even small areas of infarction. It has been accepted that patients with acute coronary syndrome (ACS) with normal CK-MB (the previous gold standard) may have elevation of troponin reflecting minimal myocardial necrosis, and are thus classified as having sustained a myocardial infarction. Furthermore elevated troponin or CK-MB predicts future cardiovascular events, underlining the role of biomarkers of necrosis in prognostication of patients. Sampling timing is however important. The release kinetics of troponin after myocardial injury and subsequent necrosis make it an unfavourable early marker of acute coronary syndrome, with maximal sensitivity only reached after at least 6 hours. Conventionally in the UK a 12 hour post chest pain sample is considered acceptable. The development of highly sensitive troponin (hsTn) assays has improved the diagnostic accuracy of troponin measurements at presentation in comparison to standard troponin assays (Reichlin *et al.,* 2009). However this improved sensitivity is coupled with a significant reduction in specificity compared to standard troponin assays, leading to a higher number of false positive results.

As a significant proportion of patients will present before 6 hours after symptoms occur, there has been extensive research interest in early biomarkers of myocardial necrosis. Heart fatty acid binding protein (H-FABP) has been prospectively investigated in this regard. H-FABP is a small protein (14-15 kDa) and as such is released from cardiac myocytes much quicker than troponin. Detectable levels occur 2-3 hours after myocardial injury and return to baseline within 12-24 hours. In combination with 4th generation troponin assays H-FABP levels have been shown to improve diagnosis of acute coronary syndrome (McCann *et al.,* 2008) and predict prognosis independently of troponin (Kilkullen *et al.,* 2007).

There remains a clinical need for a strategy to better enable rapid diagnosis and risk stratification when a patient presents with chest pain. A quick and accurate diagnosis/risk stratification would mean that high risk patients can be assigned to the appropriate treatment pathway and low risk patients can be reassured that they have not suffered an AMI and alternative causes of chest pain can be investigated. The current strategy often requires continued monitoring over a period of up to 12 hours from admission along with serial blood sampling. Such a delay in confirming diagnosis of AMI not only increases the risk of complications but also contributes to overcrowding and related costs in the emergency department. As described herein the current invention enables a method to improve early diagnosis and risk stratification of AMI in patients presenting with chest pain.

## References

Bathia et al. (2009) Annals of Clinical Biochemistry; 46(6):464-467.

Cury et al. (2008) Circulation; 118(8):837-844.

Kilcullen, N, et al. (2007) Journal of the American College of Cardiology; 50:2061-2067.

McCann, C, et al. (2008) European Heart Journal; 29:2843-2850.

McClelland et al. (2003) The American Journal of Cardiology; 92(3):252-257.

Owens et al. (2004) Journal of Electrocardiology; 37:223-232.

Pope et al. (2000) The New England Journal of Medicine; 342: 1163-1170.

Reichlin et al. (2009) The New England Journal of Medicine; 361(9):858-867.

Speake and Terry (2001) Emergency Medical Journal; 18(1):61-62.

Storrow and Gibler (2000) Annals of Emergency Medicine; 39(5): 449-461.

Thygesen et al. (2012) Nature Reviews Cardiology; 9:620-633.

Dinh, W. et al (2011) BMC Cardiovascular Disorders, BIOMED CENTRAL, 1471-2261,

## Brief Description of the Drawings

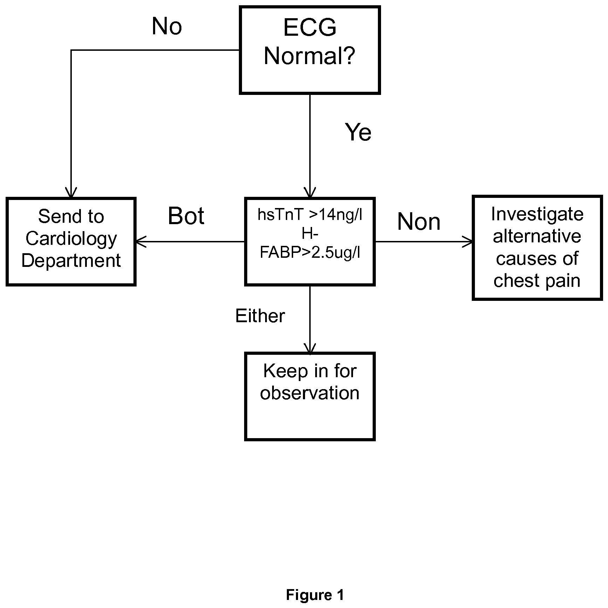

Figure 1 - Summary flow chart showing an example of how the current invention could be utilised in the emergency room.

## Summary of the Invention

The current invention demonstrates that a combination of an ECG, cardiac troponin level and H-FABP level on admission can be used to risk stratify and aid in the diagnosis of patients presenting with chest pain believed to be ischaemic in nature.

## Detailed Description

Acute coronary syndrome (ACS) refers to a set of signs and symptoms usually a combination of chest pain and other features interpreted as being a result of abruptly decreased blood flow to the heart (cardiac ischemia). The subtypes of ACS include unstable angina (UA, not associated with heart muscle damage) and two forms of myocardial infarction (non-ST & ST elevated) in which heart muscle is damaged.

Acute myocardial infarction (AMI or MI), more commonly known as a heart attack, refers to a medical condition that occurs when the blood supply to a part of the heart is interrupted, most commonly due to rupture of a vulnerable plaque. The resulting ischemia (oxygen shortage) if left untreated for a sufficient period can cause damage and/or death to heart tissue.

The present invention provides a method of risk stratification of a subject presenting with chest pain thought to be ischaemic in nature. The method of the present invention is based on the outcome of three factors determined on admission; Electrocardiogram, cardiac troponin level and H-FABP level. Dependent on the results of these three tests the subject can be placed into an appropriate risk category for an AMI having recently occurred and the appropriate treatment pathway can be initiated.

Wherein ECG, cardiac troponin and H-FABP are all negative, the subject can be placed into a low risk category for an AMI having recently occurred and other causes of chest pain can be investigated. Wherein the ECG and cardiac troponin are negative but the H-FABP is positive the subject can be placed into a moderate risk category for an AMI having recently occurred and they can be scheduled for further assessment. In cases wherein the ECG is negative but both cardiac troponin and H-FABP are positive the subject can be placed into a high risk category for an AMI having recently occurred and can be referred immediately to a cardiologist for treatment or intervention. Wherein the ECG is positive either on its own or in combination with a positive result for cardiac troponin and/or H-FABP the subject is also placed into a high risk category for an AMI having recently occurred and can be referred immediately to a cardiologist for treatment or intervention.

The term 'H-FABP' as used herein refers to heart fatty acid-binding protein, also known as fatty acid binding protein 3 (FABP-3) and fragments or partial peptides thereof, which in humans is encoded on the *FABP3* gene. H-FABP is present in the myocardium and is believed to be rapidly released into circulation as a result of myocardial injury. Numerous studies have demonstrated that it is an early marker for occurrence of MI. It will be understood to those skilled in the art that H-FABP incorporates any variant polypeptides which share the same essential biological and immunological properties as the specific H-FABP peptides used in the current invention.

The terms 'troponin' (Tn) and 'cardiac troponin' (cTn) as used by the current invention refer to all isoforms of troponin which are expressed in the cells of the heart, preferably they refer to cardiac troponin T or cardiac troponin I and fragments or partial peptides thereof, most preferably they refer to cardiac troponin T (cTnT).

The terms 'cardiac troponin level' and 'H-FABP level' refer to the concentration of cTnT and H-FABP respectively, preferably their concentration in a plasma or serum sample from a subject of the current invention. The 'reference values' or 'cut-off levels' used in the current invention are commonly acceptable levels of cTnT and H-FABP which are indicative of acute myocardial infarction. For the purposes of the current invention any value less than these reference levels may be referred to as a 'negative' test, while any value greater than these reference levels may be referred to as a 'positive' test. Preferably for cTnT the reference value is based upon the 99th percentile of a reference population as determined by the manufacturer. In the current invention the preferred cut-off level used for cTnT was 0.014ng/ml and any cTnT value below this reference value indicated that a recent AMI has not occurred. Those skilled in the art will understand that the cut-off level can vary depending on the manufacturer of the kit used to detect a biomarker. Therefore any cut-off levels recommended in a high sensitivity troponin T assay can fall under the scope of the current invention. While cTnT is the preferred cardiac troponin of the invention, any cardiac troponin which could be an indicator of tissue damage may also be used such as cardiac troponin I. Wherein the cardiac troponin is cardiac troponin I (cTnI) the preferred cut-off value for ruling out a myocardial infraction is based on the 99th percentile of a reference population as determined by the assay manufacturer, and a cTnI value less than this reference value is considered a negative result and indicates that a recent AMI has not occurred. Preferably for H-FABP the reference value is based on the 95th percentile of a reference population as determined by the manufacturer. For the purposes of the current invention the reference value for H-FABP was 2.5ng/ml. Any H-FABP value below this value is considered a negative result and indicates that a recent AMI has not occurred. Those skilled in the art will recognise that alternative H-FABP cut-off values recommended for ruling out acute myocardial infarction could also be used.

In the context of the current invention the phrase 'positive' or 'abnormal' ECG refers to any ECG reading which has ECG features or characteristics which are commonly associated with an acute myocardial infarction. These may include for example ST segment elevation or new onset left bundle branch block or ST segment depression or T wave inversion. A 'negative' or 'normal' ECG as referred to herein indicates an ECG reading devoid of such features or characteristics listed above. The ECG is preferably a standard 12-lead ECG but any suitable variation known to those skilled in the art may be used to interpret the electrical activity of the heart. The ECG may be a paramedic ECG taken en route to the hospital or medical facility or an emergency department ECG.

The term 'ruling out' as used herein refers to excluding a diagnosis of myocardial infarction in a subject presenting with chest pain. Any patient in whom AMI cannot be ruled out by the combination of the current invention can be admitted and fast-tracked for further tests or treatment while those ruled out can be assessed for alternative causes of chest pain.

A 'subject' or 'patient' of the current invention is an animal, more preferably a mammal, even more preferably a human. The subject is most preferably one whom presents to an emergency department with chest pain but can refer to any suspected, assumed or possible ACS case in which symptoms are present but no diagnosis has been established. The sample from said subjects of the current invention can be selected from a body fluid, separated cells or tissue sample. Most preferably the sample is a body fluid, even more preferably the sample is serum or plasma. In a preferred embodiment of the current invention the in vitro determinations of H-FABP and cardiac troponin are carried out by immunoassay using any means known in the art. In another preferred embodiment the immunoassay is incorporated into an automated analyser device.

A further aspect of the current invention was the novel finding that H-FABP measurements at 3 hours post-admission strongly correlated with troponin measurements at 12 hours. This could be applicable for patients who present unusually early and have negative results for ECG, troponin and H-FABP on admission. In these patients it could save the need for lengthy waits on serial troponin measurements and enable a more rapid diagnosis.

While the current invention provides a novel method for risk stratification of chest pain patients and can aid in a more rapid and efficient diagnosis of acute myocardial infarction it will be understood that clinical assessment by attending physicians will also form an important part of the diagnosis and initiation of effective and appropriate medical treatment and management. As such the current invention could be incorporated into currently used risk algorithms such as thrombolysis in myocardial infarction (TIMI) or Global registry of acute coronary events (GRACE). It is envisaged that in the future the risk stratification method provided by the current invention could be embodied by a point-of-care device that rapidly processes the results of ECG, H-FABP and cardiac troponin measurements and provides the physician with a simple and accurate risk output.

**Methods**

**Patient recruitment**

Patients presenting to the accident and emergency department of Craigavon Area Hospital, Northern Ireland, with chest pain of presumed ischaemic origin were enrolled from October 2009 until the end of October 2011. From May 2010 until June 2011, patients presenting to Daisy Hill Hospital, Newry, Northern Ireland were also recruited. In total 609 patients were recruited. Patients were excluded from the final analysis for the following reasons: duplicate recruitment, recruited greater than 12 hours after symptom onset, withdrawal of consent, chronic anticoagulant therapy, unusable serum/plasma samples and incorrect sample timing. This resulted in an overall cohort of 548 patients, 534 recruited from Craigavon Area Hospital and 14 from Daisy Hill Hospital, Newry eligible for final analysis (Figure 1). Of these patients 155 (28.3%) had a final adjudicated diagnosis of MI, 86 (15.7%) of ACS and 307 (56.0%) of non-ischaemic chest pain. Of patients diagnosed with MI, 70 (45.1%) were classified as STEMI, the remaining 85 (54.8%) MI's classified as NSTEMI. Patients with a final diagnosis of MI were more likely to be older, male, have a higher admission systolic blood pressure (BP) and be current smokers. Patients with MI were also more likely to present earlier after symptom onset, resulting in a shorter symptom onset to first blood draw time (235 minutes vs. 290 minutes non ischaemic chest pain vs. 323 minutes ACS, $p<0.001$). Patients who presented early and had a symptom onset to first blood draw time of less than 360 minutes were more likely to have ST segment elevation on an initial ECG and have a final adjudicated diagnosis of MI. The majority of patients had the first blood sample taken less 360 minutes after symptom onset (n=359, 66%, mean 215min, SD 79).

**Sample collection and preparation**

Protocol-mandated venesection occurred at admission and then subsequently at 1, 2, 3, 6, 12 and 24 hours. A leeway of 10 minutes either side of sample times at 1, 2, 3 and 6 hours and 30 minutes either side of sample times at 12 and 24 hours was permissible. At each blood draw approximately 20mls of blood was collected into 2 x 4ml serum separator gel tubes, 1 x 4ml ethylenediaminetetraacetic acid (EDTA) tube, 1 x 2.7 ml sodium citrate tube and 1 x 3 ml lithium heparin gel separator tube (all Vacutainer® Franklin Lakes, NJ, USA). Samples were immediately centrifuged at 3500 rpm for 5 minutes and subsequently transferred in $500\mu l$ aliquots into 2ml cryo-tubes (Sarstedt Ltd, Leicester, UK), and frozen at - 80°C.

**ECG analysis**

Blinded 12 lead ECG analysis was performed by the inventors. The initial presenting ECG was analysed. Presence of the following ECG features in each lead was noted:

- ST segment elevation of 1mm at the J-point
- ST segment elevation of 2mm at the J-point
- ST segment depression of 0.5mm 80ms after the J-point
- Q waves of ≥0.03ms wide and ≥25% of the size of the following R wave
- T wave inversion of at least 1mm
- Left bundle branch block
- Right bundle branch block
- Atrial fibrillation
- Paced rhythm
- Presence of left ventricular hypertrophy (Cornell criteria, (183)).

**Biomarker analysis**

Prior to analysis, aliquots were thawed on a mechanised roller for at least 20 minutes at room temperature. Once biomarker analysis had been performed, any residual sample aliquot was discarded. Biomarker analysis was only performed on samples having undergone 1 freeze thaw cycle. H-FABP concentration was measured using a cardiac biochip according to the manufacturer's instructions for use (Randox Laboratories, Crumlin, Northern Ireland). This is a solid state commercially available method based on the two step sandwich chemiluminescent immunoassay principle.

The biochip assays were performed as follows:

1. Two vials of calibrator and control reconstitution materials were reconstituted with double distilled water and placed on a mechanised roller for 10 minutes to ensure adequate mixing.

2. Nine vials of lypophilised calibrator material and 3 vials of lypophilised control material were reconstituted with the solution from step one. Once reconstituted these vials were placed on a mechanised roller for 10 minutes.

3. 240 $\mu$l of 19mMol tris-(hydroxymethyl)-aminomethane (TRIS) buffered saline, pH 7.0 containing protein surfactant, preservatives and blocking agents was pipetted onto each of the nine wells of the cardiac biochip array. Micropipetted on to this was 60 $\mu$l of sample, calibrator or control material. The biochips were then placed onto a thermoshaker device, and incubated at 37°C for 30 minutes at 380 rpm.

4. Following incubation, biochips were emptied of their reagent mixture. Adding approximately 350 $\mu$l TRIS buffered saline, pH 7.4, to each sample well, two quick washes and 4 x 2 minute soaks were performed.

5. Following the wash cycles, biochips were tapped dry of surface wash solution on lint free paper.

6. 300 $\mu$l of 19mM TRIS buffered saline, pH 7.0 with protein surfactant, stabilizers, preservatives, blocking agents and assay specific horseradish peroxidise (HRP) was then pipetted into each sample well. This was placed on the thermoshaker and incubated at 37°C for 30 minutes at 380 rpm.

7. Following incubation a further wash cycle identical to the process in step 4 was performed.

8. To prevent sample wells drying buffered wash solution was left in each well until analysis.

9. The biochip was tapped dry on lint free paper. 250 $\mu$l of signal reagent (1:1 solution luminol and peroxide) was then added to each well and the biochip placed into the carrier of the Evidence Investigator imaging machine exactly 2 minutes +/- 10 seconds after the addition of signal reagent. The Evidence Investigator takes two high resolution pictures of the biochip. Automated quantification of sample fluorescence (and hence biomarker concentration) is then performed. In cases of software failure, images can be manually analysed.

The reference range for the H-FABP assay has been determined by an independent group, demonstrating a 99th centile upper limit of 5.3 $\mu$g/L and 5.8 $\mu$g/L in females and males respectively (Bathia et al 2009). This gender difference was not statistically significant. In this study decision thresholds of 5.3 $\mu$g/L and 2.5 $\mu$g/L (95th centile) were chosen for both male and female subjects. Calibration and control runs were performed to the manufacturers recommendations.

4th generation troponin T and highly sensitive troponin T were analysed with a Roche E170 modular analyser (Roche Diagnostics, Basel, Switzerland). The 99th centile for the hsTnT assay was derived from a cohort of 533 healthy volunteers (14ng/L CI 12.7-24.9 ng/L) (Roche package insert). The 99th centile of 10 ng/L for the 4th generation troponin T assay was derived from a cohort of 1951 healthy volunteers. A CV of $\leq$ 10% was only achievable at a concentration of 30ng/L (Roche package insert) and therefore this was chosen as the diagnostic threshold for the 4th generation troponin assay.

## Final Diagnosis

The final diagnosis for each patient in the study was made by the inventors, blinded to the results of investigational biomarkers. A random sample of 50 patients was chosen and diagnosis was adjudicated by an independent physician. In cases where opinions on final diagnosis diverged, a third independent adjudicating physician made the final diagnosis. Diagnostic categories were assigned using the following criteria:

### Acute Myocardial Infarction

Diagnosis of MI was made according to the 2007 universal definition of MI. An upper cut off using a 4th generation troponin T assay (Roche Diagnostics, Burgess Hill, UK) of 0.03 $\mu$g/L was chosen, representing the lowest level at which the co-efficient of variance of the assay was 10%. The limit of detection with this assay is 0.01 $\mu$g/L.

ST-segment elevation MI (STEMI) was diagnosed when ST segment elevation at the J-point was > 1mm in ECG leads I-III, aVL, aVF, V4-V6 and > 2mm in leads V1-V3. Non-ST segment elevation MI (NSTEMI) was diagnosed through the exclusion of STEMI.

Acute Coronary Syndrome

For the purposes of classification for this investigation, acute coronary syndrome was defined as recent onset (<2 weeks) ischaemic chest pain at rest or with minimal exertion and 4th generation troponin levels that did not meet the criteria for MI. In addition there had to be either:

1. ECG ST segment depression of at least 0.5 mm 80ms after the J-point or T wave inversion of $\geq$ 1 mm at the nadir (or pseudo normalisation of resting T wave inversion) associated with typical symptoms.
Or

2. Evidence of myocardial ischaemia on functional testing, i.e. positive exercise stress test, inducible perfusion defect with stress myocardial perfusion imaging or regional myocardial hypokinesis on dobutamine stress echocardiography.
Or

3. Obstructive ($\geq$ 70% stenois) coronary artery disease in at least one epicardial coronary artery on invasive angiography.

Non-Ischaemic chest pain

Following the exclusion of MI and ACS, non-ischaemic chest pain was diagnosed.

## Results

Table 1 details the probability of acute myocardial infarction for each potential result of the biomarkers individually or in combination with each other. It was identified that there is several potential patient treatment pathways and stratifying patients based on the results could allow you to make a decision on the pathway they should follow. In this, if the probability of a patient with a specific result pattern of having an MI is below 5%, it was deemed that for these patients it would be safe to rule out AMI and other causes of chest pain can be investigated (based on an admission sample). In the optimal model, this would be a negative result for ECG, hsTnT and H-FABP, of these patients 1.6% would have been identified incorrectly (these were noted as having unusual clinical presentations). The highest risk category was defined by having the highest probability of MI. Current practice would indicate that all patients with a positive ECG reading would be referred to a cardiologist, the remainder of patients can be classified based on their biomarker results. The odds ratio provides a summary of what a specific result means in accordance to the odds of that patient having had an MI. This is calculated as follows;

$$Odds\ Ratio = \frac{\left(\frac{P_{mi}}{P_{non-mi}}\right)_{tested}}{\left(\frac{P_{mi}}{P_{non-mi}}\right)_{total}}$$

Where Pmi and Pnon-mi are the probability of having an MI and the probability of not having an MI in the tested (population with the given result) and total population (all patients regardless of the test result).

Under the method of the current invention any patient with a positive ECG would be referred immediately to a cardiologist and those with a negative ECG can be risk stratified depending on the outcome of the biomarker assays. Table 2 shows the probabilities of MI and associated odds ratios for ECG negative patients dependent on the outcome of H-FABP and TnT measured on admission. Table 3 shows the results of a Pearson correlation performed to determine if there was a relationship between H-FABP levels and TnT levels at different time points. It was found that H-FABP measurements at 3 hours from admission were strongly correlated with troponin T measurements at 6 and 12 hours form admission (R= 0.845 and 0.863 respectively).

**Table 1** The probability of acute myocardial infarction for each potential result of the biomarkers individually or in combination with each other and associated odds ratio for each.

| | Biomarker Combinations | MI | Non-MI | Total | Prob of MI | Prob of Non-MI | Odds Ratio |
|---|---|---|---|---|---|---|---|
| | ECG (-) | 84 | 376 | 460 | 0.183 | 0.817 | 1.040 |

(continued)

| | Biomarker Combinations | MI | Non-MI | Total | Prob of MI | Prob of Non-MI | Odds Ratio |
|---|---|---|---|---|---|---|---|
| ECG (-) | ECG (+) | 71 | 16 | 87 | 0.816 | 0.184 | 20.662 |
| | HFABP (-) | 11 | 253 | 264 | 0.042 | 0.958 | 0.200 |
| | HFABP (+) | 67 | 106 | 173 | 0.387 | 0.613 | 2.909 |
| | ECG (-) & HFABP (-) | 11 | 253 | 264 | 0.042 | 0.958 | 0.202 |
| | ECG (-) & HFABP (+) | 67 | 106 | 173 | 0.387 | 0.613 | 2.943 |
| | ECG (+) & HFABP (-) | 9 | 10 | 19 | 0.474 | 0.526 | 4.191 |
| | ECG (+) & HFABP (+) | 60 | 4 | 64 | 0.938 | 0.063 | 69.844 |
| ECG (-) | hsTnT (-) | 7 | 238 | 245 | 0.029 | 0.971 | 0.134 |
| | hsTnT (+) | 61 | 72 | 133 | 0.459 | 0.541 | 3.862 |
| | ECG (-) & hsTnT (-) | 7 | 238 | 245 | 0.029 | 0.971 | 0.137 |
| | ECG (-) & hsTnT (+) | 61 | 72 | 133 | 0.459 | 0.541 | 3.945 |
| | ECG (+) & hsTnT (-) | 8 | 7 | 15 | 0.533 | 0.467 | 5.321 |
| | ECG (+) & hsTnT (+) | 49 | 3 | 52 | 0.942 | 0.058 | 76.052 |
| ECG (-) | HFABP (-) & hsTnT (-) | 3 | 183 | 186 | 0.016 | 0.984 | 0.076 |
| | HFABP (+) & hsTnT (-) | 3 | 45 | 48 | 0.063 | 0.938 | 0.310 |
| | HFABP (-) & hsTnT (+) | 7 | 29 | 36 | 0.194 | 0.806 | 1.124 |
| | HFABP (+) & hsTnT (+) | 51 | 41 | 92 | 0.554 | 0.446 | 5.792 |
| | ECG (-) & HFABP (-) & hsTnT (-) | 3 | 183 | 186 | 0.016 | 0.984 | 0.042 |
| | ECG (-) & HFABP (+) & hsTnT (-) | 3 | 45 | 48 | 0.063 | 0.938 | 0.171 |
| | ECG (-) & HFABP (-) & hsTnT (+) | 7 | 29 | 36 | 0.194 | 0.806 | 0.620 |
| | ECG (-) & HFABP (+) & hsTnT (+) | 51 | 41 | 92 | 0.554 | 0.446 | 3.193 |
| ECG (+) & HFABP (-) & hsTnT (-) | | 5 | 7 | 12 | 0.417 | 0.583 | 1.833 |
| ECG (+) & HFABP (+) & hsTnT (-) | | 3 | 0 | 3 | 1.000 | 0.000 | Not Calc |
| ECG (+) & HFABP (-) & hsTnT (+) | | 3 | 1 | 4 | 0.750 | 0.250 | 7.700 |
| ECG (+) & HFABP (+) & hsTnT (+) | | 45 | 2 | 47 | 0.957 | 0.043 | 57.750 |

**Table 2** Probability of acute myocardial infarction and odds ratios for ECG negative patients based on the combination of their H-FABP and troponin T results.

| Biomarker Combinations | MI | Non-MI | Total | Prob of MI | Prob of Non-MI | Odds Ratio |
|---|---|---|---|---|---|---|
| HFABP (-) & hsTnT (-) | 3 | 183 | 186 | 0.016 | 0.984 | 0.076 |
| HFABP (+) & hsTnT (-) OR HFABP (-) & hsTnT (+) | 10 | 74 | 84 | 0.119 | 0.881 | 0.629 |
| HFABP (+) & hsTnT (+) | 51 | 41 | 92 | 0.554 | 0.446 | 5.792 |

**Table 3** Pearson Correlation Coefficients between H-FABP measurements at admission, 1 hr, 2hr, 3hr and highly sensitive Troponin T measurements at admission, 1 hr, 2 hr, 3 hr, 6hr and 12 hr.

| | New_ hsTnTAdmission | New_ hsTnT1 | New_ hsTnT2 | New_ hsTnT3 | New_ hsTnT6 | New_ hsTnT12 |
|---|---|---|---|---|---|---|
| FABPAdmission | .578 | .667 | .552 | .585 | .576 | .457 |

(continued)

| | New_ hsTnTAdmission | New_ hsTnT1 | New_ hsTnT2 | New_ hsTnT3 | New_ hsTnT6 | New_ hsTnT12 |
|---|---|---|---|---|---|---|
| FABP1 | .401 | .664 | .653 | .780 | .797 | .809 |
| FABP2 | .408 | .671 | .669 | .737 | .767 | .806 |
| FABP3 | .388 | .625 | .685 | .734 | .845 | .863 |

## Claims

1. A method of risk stratification of a subject presenting with chest pain suspected to be ischaemic in nature, wherein said subject has a negative ECG, said method comprising determining the levels of a cardiac troponin and H-FABP in an *in vitro* sample taken from the subject at presentation wherein the reference value for a cardiac troponin for ruling out an acute myocardial infarction (AMI) is the 99th percentile of a reference population, and wherein the reference value for H-FABP for ruling out an AMI is the 95th percentile of a reference population , and based on these results calculating the subject's probability of having suffered an AMI and risk stratifying said patient accordingly.

2. The method of claim 1 wherein if cardiac troponin and H-FABP are both negative the subject is placed into a low risk category for acute myocardial infarction.

3. The method of Claim 2 wherein an additional step comprises the determination of the levels of H-FABP in an additional in vitro sample taken from the subject 3hrs after presentation.

4. The method of claim 1 wherein if one of cardiac troponin or H-FABP is positive the subject is placed into a moderate risk category for acute myocardial infarction.

5. The method of claim 1 wherein if cardiac troponin and H-FABP are both positive the subject is placed into a high risk category for acute myocardial infarction.

6. The method of any preceding claim wherein the reference value for H-FABP for ruling out a myocardial infarction is about 2.5ng/ml and an H-FABP value less than this reference value is considered a negative result and indicates that acute myocardial infarction has not occurred.

7. The method of any preceding claim wherein the cardiac troponin is cardiac troponin T (cTnT) and the reference value for ruling out a myocardial infarction is about 0.014ng/ml and a cTnT value less than this reference value is considered a negative result and indicates that acute myocardial infarction has not occurred.

8. The method of any of claims 1-6 wherein the cardiac troponin is cardiac troponin I (cTnl)

## Patentansprüche

1. Verfahren zur Risikostratifizierung eines Patienten mit Brustschmerz, von dem vermutet wird, dass er ischämisch ist, wobei der Patient ein negatives EKG aufweist, wobei das Verfahren das Bestimmen der Konzentrationen eines Herztroponins und von H-FABP in einer in vitro entnommenen Probe umfasst das Subjekt bei der Präsentation, wobei der Referenzwert für ein Herztroponin zum Ausschluss eines akuten Myokardinfarkts (AMI) das 99. Perzentil einer Referenzpopulation ist, und wobei der Referenzwert für H-FABP zum Ausschluss eines AMI das 95. Perzentil von ist einer Referenzpopulation, und basierend auf diesen Ergebnissen, Berechnen der Wahrscheinlichkeit eines Subjekts, einen AMI erlitten zu haben, und Risiko, den Patienten entsprechend zu stratifizieren.

2. Verfahren nach Anspruch 1, wobei, wenn sowohl Herztroponin als auch H-FABP beide negativ sind, der Patient in eine Kategorie mit niedrigem Risiko für einen akuten Myokardinfarkt eingestuft wird.

3. Verfahren nach Anspruch 2, wobei ein zusätzlicher Schritt die Bestimmung der H-FABP-Konzentrationen in einer zusätzlichen In-vitro-Probe umfasst, die 3 Stunden nach der Präsentation von dem Subjekt genommen wurde.

**4.** Verfahren nach Anspruch 1, wobei, wenn eines von Herztroponin oder H-FABP positiv ist, der Patient in eine Kategorie mit mäßigem Risiko für einen akuten Herzinfarkt eingeordnet wird.

**5.** Verfahren nach Anspruch 1, wobei, wenn sowohl Herztroponin als auch H-FABP beide positiv sind, der Patient in eine Kategorie mit hohem Risiko für einen akuten Myokardinfarkt eingestuft wird.

**6.** Verfahren nach einem der vorhergehenden Ansprüche, wobei der Referenzwert für H-FABP zum Ausschluss eines Myokardinfarkts etwa 2,5 ng / ml beträgt und ein H-FABP-Wert, der niedriger als dieser Referenzwert ist, als negatives Ergebnis angesehen wird und einen akuten Myokardinfarkt anzeigt hat nicht stattgefunden.

**7.** Verfahren nach einem der vorhergehenden Ansprüche, wobei das Herztroponin Herztroponin T (cTnT) ist und der Referenzwert zum Ausschluss eines Myokardinfarkts etwa 0,014 ng / ml beträgt und ein cTnT-Wert unterhalb dieses Referenzwerts als negatives Ergebnis betrachtet wird zeigt an, dass kein akuter Herzinfarkt aufgetreten ist.

**8.** Verfahren nach einem der Ansprüche 1 bis 6, wobei das Herztroponin Herztroponin I (cTnI) ist.

**Revendications**

**1.** Méthode de stratification du risque d'un sujet présentant une douleur thoracique suspectée d'être de nature ischémique, dans laquelle ledit sujet présente un ECG négatif, ladite méthode consistant à déterminer les niveaux d'une troponine cardiaque et de H-FABP dans un échantillon in vitro prélevé dans le sujet à la présentation dans lequel la valeur de référence d'une troponine cardiaque pour exclure un infarctus aigu du myocarde (IAM) est le 99e centile d'une population de référence, et dans laquelle la valeur de référence pour H-FABP pour exclure un IAM est le 95e centile de une population de référence et, sur la base de ces résultats, calcule la probabilité que le sujet soit atteint d'un IAM et risque de stratifier ledit patient en conséquence.

**2.** Procédé selon la revendication 1, dans lequel si la troponine cardiaque et la H-FABP sont toutes deux négatives, le sujet est classé dans une catégorie à faible risque d'infarctus aigu du myocarde.

**3.** Procédé selon la revendication 2, dans lequel une étape supplémentaire comprend la détermination des niveaux de H-FABP dans un échantillon supplémentaire in vitro prélevé sur le sujet 3 heures après la présentation.

**4.** Procédé selon la revendication 1, dans lequel, si une des troponines cardiaques ou H-FABP est positive, le sujet est placé dans une catégorie de risque modéré d'infarctus aigu du myocarde.

**5.** Procédé selon la revendication 1, dans lequel, si la troponine cardiaque et la H-FABP sont toutes deux positives, le sujet est classé dans une catégorie à haut risque d'infarctus aigu du myocarde.

**6.** Procédé selon l'une quelconque des revendications précédentes, dans lequel la valeur de référence de H-FABP pour éliminer un infarctus du myocarde est d'environ 2,5 ng / ml et une valeur de H-FABP inférieure à cette valeur de référence est considérée comme un résultat négatif et indique qu'un infarctus aigu du myocarde n'a pas eu lieu.

**7.** Procédé selon l'une quelconque des revendications précédentes, dans lequel la troponine cardiaque est la troponine cardiaque T (cTnT) et la valeur de référence pour exclure un infarctus du myocarde est d'environ 0,014ng / ml et une valeur de cTnT inférieure à cette valeur de référence est considérée comme un résultat négatif. indique qu'il n'y a pas eu d'infarctus aigu du myocarde.

**8.** Procédé selon l'une quelconque des revendications 1 à 6, dans lequel la troponine cardiaque est la troponine cardiaque I (cTnI).

No      ┌─────────────┐
                  │ ECG Normal? │
                  └─────────────┘

Ye

| Send to Cardiology Department | Bot | hsTnT >14ng/l H-FABP>2.5ug/l | Non | Investigate alternative causes of chest pain |

Either

Keep in for observation

Figure 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BATHIA et al.** *Annals of Clinical Biochemistry,* 2009, vol. 46 (6), 464-467
- **CURY et al.** *Circulation,* 2008, vol. 118 (8), 837-844
- **KILCULLEN, N et al.** *Journal of the American College of Cardiology,* 2007, vol. 50, 2061-2067
- **MCCANN, C et al.** *European Heart Journal,* 2008, vol. 29, 2843-2850
- **MCCLELLAND et al.** *The American Journal of Cardiology,* 2003, vol. 92 (3), 252-257
- **OWENS et al.** *Journal of Electrocardiology,* 2004, vol. 37, 223-232
- **POPE et al.** *The New England Journal of Medicine,* 2000, vol. 342, 1163-1170
- **REICHLIN et al.** *The New England Journal of Medicine,* 2009, vol. 361 (9), 858-867
- **SPEAKE ; TERRY.** *Emergency Medical Journal,* 2001, vol. 18 (1), 61-62
- **STORROW ; GIBLER.** *Annals of Emergency Medicine,* 2000, vol. 39 (5), 449-461
- **THYGESEN et al.** *Nature Reviews Cardiology,* 2012, vol. 9, 620-633
- **DINH, W. et al.** BMC Cardiovascular Disorders. BIOMED CENTRAL, 2011, 1471-2261